(19) Europäisches Patentamt
European Patent Office
Office européen des brevets

(11) **EP 4 751 728 A1**

(12) **EUROPEAN PATENT APPLICATION**
published in accordance with Art. 153(4) EPC

(43) Date of publication:
03.06.2026 Bulletin 2026/23

(21) Application number: 24844632.0

(22) Date of filing: 11.07.2024

(51) International Patent Classification (IPC):
*A61K 36/79* (2006.01)    *A61K 36/57* (2006.01)
*A61K 36/233* (2006.01)    *A61P 1/16* (2006.01)
*A61P 3/06* (2006.01)

(86) International application number:
PCT/CN2024/104919

(87) International publication number:
WO 2025/020939 (30.01.2025 Gazette 2025/05)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 25.07.2023 CN 202310919939
07.03.2024 CN 202410263883

(71) Applicant: Tsing Hua De Ren Xi'An Xingfu
Pharmaceutical
Co. Ltd.
Xi'an, Shaanxi 710043 (CN)

(72) Inventor: DU, Chengqiang
Xi' an, Shaanxi 710043 (CN)

(74) Representative: Schiweck Weinzierl Koch
Patentanwälte Partnerschaft mbB
Ganghoferstraße 68 B
80339 München (DE)

(54) **USE OF WULING FORMULATION IN THE PREPARATION OF DRUG FOR PREVENTING OR TREATING NON-ALCOHOLIC FATTY LIVER DISEASE**

(57) The use of a Wuling formulation in the preparation of a medicament for preventing or treating non-alcoholic fatty liver disease. Effective medicine components of the Wuling formulation comprise radix bupleuri, glossy ganoderma, radix salviae miltiorrhizae and fructus schisandrae chinensis. Said formulation has simple components, but the four medicines are synergistic and play an effective role in preventing or treating non-alcoholic fatty liver disease.

Example Image

Normal Control

Model Control

Atorvastatin Calcium 11.6 µg/mL

Wuling Capsule 12.5 µg/mL

Wuling Capsule 25.0 µg/mL

Wuling Capsule 50.0 µg/mL

Figure 1

## Description

### Cross-reference to Related Applications

The present application claims the benefit of priorities of CN Patent Application No. 202310919939.7 entitle "Use of Wuling Formulation in the Preparation of Drug for Preventing or Treating Non-Alcoholic Fatty Liver Disease" filed on 25 July 2023 and CN Patent Application No. 202410263883.9 entitled "Use of Wuling Formulation in the Preparation of Drug for Preventing Or Treating Non-Alcoholic Fatty Liver Disease" filed on 7 March 2024, the contents of which are hereby incorporated by reference in their entirety for all purposes.

### Field of the Invention

The present invention relates to the field of traditional Chinese medicines, in particular to use of Wuling formulation in the preparing a medicament for preventing or treating non-alcoholic fatty liver disease.

### Background of the Invention

Fatty liver is characterized by excessive deposition of lipids in hepatocytes. When lipid accumulation in hepatocytes exceeds 5% of liver wet weight, or histologically when steatosis is observed in more than one-third of hepatocytes per unit area, it is referred to as fatty liver disease. Non-alcoholic fatty liver disease (NAFLD) is a clinicopathological syndrome caused by factors other than alcohol and other definite liver-damaging factors, primarily characterized by diffuse hepatic macrovesicular steatosis. The accumulation of lipids in the liver is the main cause of NAFLD, with pathological manifestations including vacuolar steatosis of hepatocytes, inflammation within hepatic lobules, hepatocyte necrosis and balloon-like degeneration, and the like. With the significant rise in the global incidence of obesity and metabolic syndrome, NAFLD has now become the most common chronic metabolic liver disease worldwide and is currently the leading chronic liver disease in China.

Non-alcoholic fatty liver disease is one of the most common precursors to chronic liver diseases (such as liver fibrosis and hepatocellular carcinoma) and metabolic disorders (such as obesity, type II diabetes, and atherosclerosis). Patients with NAFLD cannot recover from this condition; instead, the disease progresses to non-alcoholic steatohepatitis (NASH), hepatic fibrosis and cirrhosis. Moreover, as a significant member of metabolic syndrome, it is closely associated with cardiovascular and cerebrovascular diseases. This severely impacts people's physical health and quality of life, and also places a heavy burden on society.

Currently, the treatment for fatty liver disease primarily involves eliminating causative factors, aggressive treatment of underlying diseases, and adherence to a balanced diet, while medicament serves only an auxiliary role. At present, the drugs clinically used for treating fatty liver disease are mainly categorized into lipid-lowering agents, hepatoprotective and lipid-modifying agents and traditional Chinese herbal medicines. However, the traditional Chinese herbal medicines often have complex components and intricate manufacturing processes.

### Summary of the Invention

The present application provides a use of a Wuling formulation in the preparation of a medicament for preventing or treating non-alcoholic fatty liver disease.

In the first aspect of the present invention, it provides the use of a Wuling formulation in the preparation of a medicament for preventing or treating non-alcoholic fatty liver disease.

In some embodiments, the Wuling formulation comprises, in parts by weight: 200 to 400 parts of Bupleuri radix (the root of *Bupleurum* species) , 100 to 200 parts of Ganoderma (the sporocarp *Ganoderma lucidum* ( Leyss. ex Fr.) Karst. or *Ganoderma sinense* Zhao, Xu et Zhang), 200 to 350 parts of Salviae miltiorrhizae radix et rhizome (the root and rhizome of *Salviae miltiorrhiza* Bge.) and 250 to 350 parts of Schisandrae fructus (the fruit of *Schisandra chinesis* (Turcz.) Baill or *Schisandra sphenanthera* Rehd. et Wils.).

In some embodiments, the Wuling formulation comprises, in parts by weight: 300 to 350 parts of Bupleuri radix, 150 to 180 parts of Ganoderma, 300 to 350 parts of Salviae miltiorrhizae radix et rhizome and 300 to 350 parts of Schisandrae fructus.

In some embodiments, the Wuling formulation comprises, in parts by weight: 342 parts of Bupleuri radix, 173 parts of Ganoderma, 342 parts of Salviae miltiorrhizae radix et rhizome and 342 parts of Schisandrae fructus.

In some embodiments, the Wuling formulation is prepared in a dosage form selected from the group consisting of a decoction, a pill, an oral liquid, a tablet, a capsule, a granule, a medicated electuary (i.e., Herbal Paste for oral use), and a powder.

In some embodiments, the Wuling formulation is selected from any one of Wuling Capsule, Wuling Pill and Wuling Powder.

In some embodiments, the Wuling formulation is administered to an adult in a unit dose of 3 g to 6 g, three times per day.

The effective components of the Wuling formulation comprise Bupleuri radix, Ganoderma, Salviae miltiorrhizae radix et rhizome and Schisandrae fructus. Despite its simple composition, the four medicinal components act synergistically to exert a therapeutic action in the prevention or treatment of non-alcoholic fatty liver disease (NAFLD).

**Brief Description of Drawings**

In order to more clearly illustrate the technical solutions of the embodiments of the present application, the figures required for describing the embodiments will be briefly introduced below. It is obvious that the figures described below relate to only some embodiments of the present application. Those skilled in the art will arrive at other figures according to these figures without any creative efforts.

FIG. 1 shows representative images of liver fat staining intensity in zebrafish treated with Wuling Capsule according to Example 1 of the present application, wherein the area within the yellow dashed line represents the liver.

FIG. 2 shows representative images of the liver tissue structure in zebrafish treated with Wuling Capsule according to Example 1 of the present application, wherein the area within the yellow dashed line indicates the analyzed zebrafish liver region, the red arrow points to hepatocellular swelling, and the black arrow points to fatty vacuolar degeneration.

**Detailed Description of the Invention**

The following examples are given to further describe the embodiments of the present application in detail. The detailed description of the following examples is intended to illustrate the principles of the present invention, but should not be construed as limiting the scope of the present application, i.e., the application is not limited to the examples described.

As analyzed in the section of Background of the present disclosure, although there have been some compositions of traditional Chinese herbal medicine for treating non-alcoholic fatty liver disease in the prior art, their components are complex. In addition, the applicant has conducted research on the medicines currently used in clinical for treating chronic hepatitis, hepatitis B, cirrhosis, hepatitis B-related liver fibrosis, compensatory cirrhosis and the like, and found that the medicines effective for the aforementioned diseases do not provide guidance for treating non-alcoholic fatty liver disease. For example, Fufang Biejia Ruangan Tablets have shown remarkable therapeutic effects for the conditions mentioned above, but exhibit no efficacy against non-alcoholic fatty liver disease.

In one embodiment of the present application, a use of a Wuling formulation in the preparation of a medicament for preventing or treating non-alcoholic fatty liver disease is provided.

The effective components of the Wuling formulation comprise Bupleuri radix , Ganoderma, Salviae miltiorrhizae radix et rhizome and Schisandrae fructus. Despite its simple composition, the four medicinal components act synergistically to exert a therapeutic action in the prevention or treatment of non-alcoholic fatty liver disease.

Bupleuri radix, with its pungent dispersing and bitter draining properties, effectively soothes liver qi and relieves liver stagnation, serving as the sovereign (or "monarch") drug in the composition. Ganoderma, is sweet in flavor and neutral in nature, has the effects of tonifying qi and strengthening the spleen, and reinforces healthy qi and supports the body's upright energy; Salviae miltiorrhizae radix et rhizome invigorates blood circulation, resolves stasis, unblocks meridians, and alleviates pain; the two ones act together as the minister (or "assistant") drug. Schisandrae fructus is sour-sweet in flavor and warm in nature, tonifies qi and nourishes yin. It can assist Ganoderma in supplementing qi and fortifying the middle jiao (spleen and stomach),at the same time can calm the mind, eliminate depression, and further prevent Bupleuri radix's dispersing and ascending action from excessively consuming qi and damaging yin. It also protects the liver and lowers transaminase. Therefore, Schisandrae fructus fulfills the role of an adjuvant and modulator (or "mediating /regulating" drug). When the four herbs are used in combination, they simultaneously regulate the liver and spleen, harmonize qi and blood, and balance consolidating and dispersing actions. Together, they work synergistically to soothe the liver and relieve stagnation, tonify qi and strengthen the spleen, and activate blood circulation to resolve stasis. Their combination can significantly improve symptoms in patients with non-alcoholic fatty liver disease, such as pain in the liver region, abdominal fullness and discomfort, fatigue and weakness, and poor appetite, while also lowering abnormal blood biochemical markers.

To further enhance the synergistic effect of the four herbal medicines, in some embodiments of the present application, the Wuling formulation preferably comprises, in parts by weight: 200 to 400 parts of Bupleuri radix, 100 to 200 parts of Ganoderma, 200 to 350 parts of Salviae miltiorrhizae radix et rhizome and 250 to 350 parts of Schisandrae fructus. More preferably, the Wuling formulation comprises, in parts by weight: 300 to 350 parts of Bupleuri radix, 150 to 180 parts of Ganoderma, 300 to 350 parts of Salviae miltiorrhizae radix et rhizome and 300 to 350 parts of Schisandrae fructus. Further preferably, the Wuling formulation comprises, in parts by weight: 342 parts of Bupleuri radix, 173 parts of Ganoderma, 342 parts of Salviae miltiorrhizae radix et rhizome and 342 parts of Schisandrae fructus.

The Wuling formulation of the present application may be used in any conventional dosage form. In some embodiments, the Wuling formulation is prepared in a dosage form selected from the group consisting of a decoction, a pill, an oral liquid, a tablet, a capsule, a granule, a medicated electuary (i.e., Herbal Paste for oral use), and a powder. The preparation processes for the aforementioned dosage forms are well established, facilitating the application of the Wuling formulation in preparing a medicament for preventing or treating the non-alcoholic fatty liver disease.

In some embodiments, the Wuling formulation is selected from any one of Wuling Capsule, Wuling Pill and Wuling Powder. The aforementioned formulations are all official preparations, which has advantage in the implementation of the application described herein. Through repeated experimental verification, the inventor of the present application has derived the following formula. Using the effective safe concentration for zebrafish described in this application, the equivalent theoretical human dose can be calculated by the following formula:

$$\text{human dose (g/time)} = [\text{concentration for zebrafish (μg/mL)} \times 6/100] \times 2$$

Based on the above conversion, in some embodiments, the Wuling formulation is administered to an adult in a unit dose of 3 g to 6 g, three times per day. The aforementioned dosage regimen demonstrates significant therapeutic efficacy against non-alcoholic fatty liver disease and exhibits minimal side effects in humans.

The beneficial effects of the present application will be further illustrated below in conjunction with examples and comparative examples. The scope of the present invention, however, is not limited to these examples.

## Example 1

The Wuling capsules used were provided by Tsing Hua De Ren Xingfu Pharmaceutical Co., Ltd..

### 1. Principle and methods of experiment

Experimental Principle: Zebrafish exhibits high similarity to human in terms of liver structure, function and genetics. Except for immunerelated Kupffer cells, zebrafish possesses all other cell types found in the human liver and perform the same functions which include bile secretion, glycogen and lipid storage, insulin response, xenobiotic and ammonia metabolism, as well as the secretion of serum proteins such as complement and coagulation factors, transferrin and albumin-like proteins. Within five days post-fertilization, the zebrafish liver becomes fully functional, allowing for comprehensive liver function assessments, which makes zebrafish an important model for studying liver diseases. Zebrafish is fed with a high-sugar and high-fat diet providing energy exceeding the fish needs. The surplus energy is stored as fat in the liver, leading to a continuous increase in hepatic lipid content, so that the fat content in the liver is continuously increased, which induces hepatocyte steatosis and ultimately results in fatty liver disease.

### Sample preparation information:

Wuling Capsule, solvent: standard dilution water.

Positive Control: Atorvastatin Calcium Tablets (hereinafter referred to as Atorvastatin Calcium), white tablets, batch No. FR7909, Pfizer Pharmaceuticals Ltd., solvent: DMSO.

### Experimental Animals:

Zebrafish were all reared in fish culture water at 28°C (water quality: 200 mg instant sea salt per 1 L of reverse osmosis water, conductivity 450-550 μS/cm, pH 6.5-8.5, hardness 50-100 mg/L CaCO$_3$). The zebrafish were bred and provided by the company's aquaculture center. The experimental animal use license number is SYXK (Zhe) 2022-0004. Housing and management are complied with the requirements of international AAALAC accreditation (certification number 001458). The IACUC ethics review number is IACUC-2023-7689-01.

### Instruments, Consumables and Reagents:

Dissecting microscopy (SZX 7, OLYMPUS, Japan);
CCD camera (Model VertA 1, Shanghai Tussen Vision Technology Ltd., China);

Precision electronic balances (Model CP 214, OHAUS, America);

6-well plate (Zhejiang Beilanbo Biotechnology Co., Ltd., China,);

Double-sided clean bench for two persons (Model SW-CJ-215 KS, Shanghai Sujing Industrial Co., Ltd., China);

Microtome (Model KD 2258, Jinhua Kedi Medical Equipment Co., Ltd., China);

Biological microscope (Model CX 31, OLYMPUS, Japan).

Dimethyl sulfoxide (DMSO, Batch No. BCCD8942, Sigma, Switzerland);

Methylcellulose (Batch No. C2004046, Shanghai Aladdin Biochemical Technology Ltd., China);

Absolute ethanol (Batch No. 20230329, Sinopharm Chemical Reagent Co., Ltd., China);

Oil Red O (Batch No. SHBN4926; Sigma, USA);

1, 2-propanediol (Batch No. 20211117, Sinopharm Chemical Reagent Co., Ltd., China);

4% Tissue Cell Fixative Solution (Batch No. 20221014, Beijing Solarbio Science & Technology Co., Ltd., China);

Xylene (Batch No. C14165111; Shanghai Macklin Biochemical Technology Co., Ltd., China);

PBS Buffer (Batch 70115000; Biosharp, China);

Mayer's Hematoxylin Staining Solution (Batch No. 20220120; Shanghai Yihe Biotechnology Co., Ltd., China);

Eosin Staining Solution (Batch No. 20220120; Shanghai Yihe Biotechnology Co., Ltd., China);

Neutral balsam (Batch No. 330A021; Solarbio, China);

High-performance embedding paraffin (Melting point 54-56°C, Batch No. 20201020; Shanghai Huayong Paraffin Co., Ltd., China)

High-performance embedding paraffin (Melting point 62-64°C, Batch No. 20210828; Shanghai Huayong Paraffin Co., Ltd., China).

The experimental procedure was as follows:

(1) Determination of the MTC for Wuling Capsule

Albino (AB) strain zebrafish with melanin allele mutation at 5 days post-fertilization (5 dpf) was randomly selected and placed in beakers, with 30 zebrafish in each beaker. Five test concentration groups of Wuling Capsule were set up, and the sample was administered to the zebrafish in microplates/beakers in form of aqueous solution. A normal control group and a model control group were also included, making a total of seven experimental groups. Each beaker contained 25 mL of solution. The test concentration groups received Wuling Capsule dissolved in water (concentrations are listed in Table 1). Except for the normal control group, all other groups were fed a high-sugar and high-fat diet in form of aqueous solution to establish a diet-induced fatty liver model in zebrafish (specifically, zebrafish was exposed to 1.5 mg/mL egg yolk powder solution during the day and 30 mg/mL glucose solution at night to establish the zebrafish diet-induced fatty liver model). After 2 days of treatment at 28 °C, the number of dead zebrafish and toxicity manifestations in each group were recorded to determine the Maximum Tolerated Concentration (MTC) of the sample. The results are recorded in Table 1.

TABLE 1 Experimental Results of Concentration Screening for the Hepatoprotective Effect of Wuling Capsule against Diet-Induced Fatty Liver (n = 30)

| Group | Concentration ($\mu$g/mL) | Mortality (n) | Mortality Rate (%) | Phenotype |
|---|---|---|---|---|
| Normal Control | - | 0 | 0 | No observable abnormalities |
| Model Control | - | 0 | 0 | No observable abnormalities |
| Test concentration | 25 | 0 | 0 | Comparable to the model control group |
| | 50 | 0 | 0 | Comparable to the model control group |
| | 100 | 6 | 20 | - |
| | 200 | 30 | 100 | - |
| | 400 | 30 | 100 | - |

Therefore, the MTC for Wuling Capsule on the zebrafish with diet-induced fatty liver is 50.0 $\mu$g/mL.

(2) Assessment of hepatic steatosis

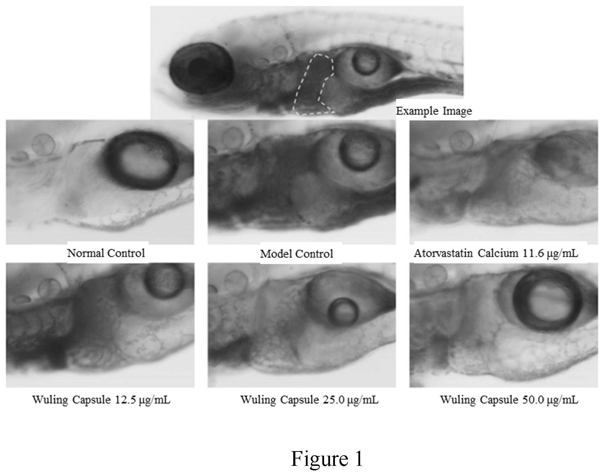

Albino (AB) strain zebrafish with melanin allele mutation at 5 days post-fertilization (5 dpf) was randomly selected and placed in beakers, with 30 zebrafish in each beaker. Wuling capsule was administered in form of aqueous solution (the concentration is shown in Table 2). Six experimental groups were established: normal control group, model control group, positive control group (Atorvastatin Calcium, 11.6 μg/mL), and three test concentration groups (MTC 50 μg/mL, 1/2 MTC 25.0 μg/mL, and 1/4 MTC 12.5 μg/mL). Each beaker contained 25 mL of solution. Except for the normal control group, all other groups were fed a high-sugar and high-fat diet in form of aqueous solution to establish a diet-induced fatty liver model in zebrafish (specifically, zebrafish was exposed to 1.5 mg/mL egg yolk powder solution during the day and 30 mg/mL glucose solution at night to establish the zebrafish diet-induced fatty liver model). After 2 days of treatment at 28 °C, the fish was subjected to whole-mount lipid staining using Oil Red O. Then, from each group, 10 zebrafish were randomly selected and photographed under a dissecting microscope. Images were acquired and analyzed using NIS-Elements D3.20 advanced image processing software to quantity hepatic staining intensity. The statistical results of this indicator were used to evaluate the auxiliary protective effect of Wuling Capsule against diet-induced fatty liver disease. Data are expressed as mean $\pm$ SE. Statistical analysis was performed using SPSS26.0 software, with $p < 0.05$ considered statistically significant. Representative images of hepatic lipid staining intensity are shown in FIG. 1, and the corresponding quantitative data are summarized in Table 2.

TABLE 2

| Group | Concentration (μg/mL) | Hepatic Staining Intensity (pixel, mean $\pm$ SE) |
|---|---|---|
| Normal control | - | 6137 $\pm$ 165*** |
| Model control | - | 24601 $\pm$ 695 |
| Positive control | 11.6 | 11925 $\pm$ 650*** |
| Test concentration | 12.5 | 19332 $\pm$ 871*** |
| | 25.0 | 12023 $\pm$ 910* |
| | 50.0 | 9243 $\pm$ 792*** |

***: p < 0.001 compared to model control group.

It is indicated that Wuling capsules have the auxiliary protective effect on the diet-induced fatty liver disease.

(3) Hepatic histopathological sections

Wild-type Albino (AB) strain zebrafish at 5 days post-fertilization (5 dpf) was randomly selected and placed in beakers, with 30 zebrafish in each beaker. All test samples were administered in form of aqueous solution. Each beaker contained 25 mL of solution. Six experimental groups were established: normal control group, model control group, positive control group (Atorvastatin Calcium, 11.6 μg/mL), and three test concentration groups (MTC 50 μg/mL, 1/2 MTC 25.0 μg/mL, and 1/4 MTC 12.5 μg/mL). Except for the normal control group, all other groups were fed a high-sugar and high-fat diet in form of aqueous solution to establish a diet-induced fatty liver model in zebrafish (specifically, zebrafish was exposed to 1.5 mg/mL egg yolk powder solution during the day and 30 mg/mL glucose solution at night to establish the zebrafish diet-induced fatty liver model). After 2 days of treatment at 28 °C, zebrafish from each group underwent fixation, dehydration, embedding, sectioning, H & E staining and the like, and then were subjected to histopathological observation and analysis.

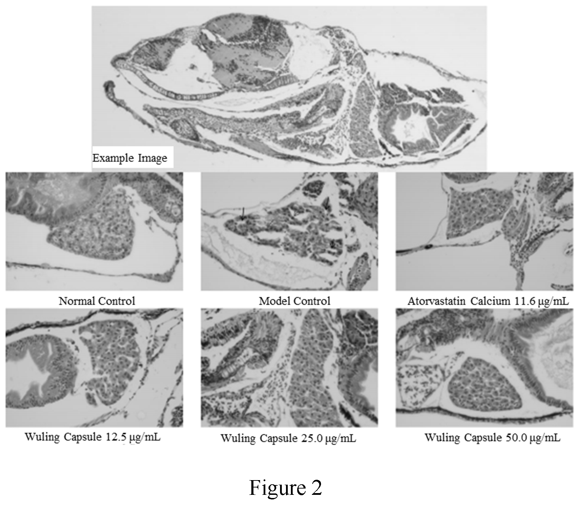

Histopathological images are shown in FIG. 2. Under the described experimental conditions, histopathological observation of zebrafish liver from the normal control group revealed regularly arranged hepatocytes, normal hepatocellular structure and clear cellular margins. Histopathological examination of zebrafish from the model control group showed blurred hepatocyte structure, swollen hepatocyte nuclei (indicated by red arrows), and fatty vacuolar degeneration in liver tissue (indicated by black arrows), which indicates that the model was successfully established. Histopathological examination of zebrafish liver from the positive control group (Atorvastatin Calcium) displayed clearer hepatocellular structure compared to the model control group, reduced hepatocyte swelling and decreased fatty vacuolation, demonstrating that Atorvastatin Calcium has an ameliorative effect on high-sugar high-fat-induced (diet-induced) fatty liver in zebrafish. Histopathological examination of the Wuling Capsule groups at concentration of 12.5, 25.0 and 50.0 μg/mL revealed clearer hepatocellular structure, diminished hepatocyte swelling and reduced fatty vacuolar degeneration in liver tissue compared to the model control group. This suggests that Wuling Capsule has an auxiliary protective effect against high-sugar high-fat-induced (diet-induced) fatty liver in zebrafish, specifically manifested as restoration of liver tissue morphology.

It should be noted that the present application is not limited to the above-described embodiments. The aforementioned embodiments are merely illustrative, and any embodiments having a configuration substantially identical to the technical concept of the present application and achieving the same effects fall within the technical scope of the present application. Furthermore, within the scope of the present application without departing from its essence, various modifications applied to the embodiments that can be conceived by those skilled in the art, as well as other implementations constructed by combining some constituent elements of the embodiments, are also included within the scope of the present application.

## Claims

1. A use of a Wuling formulation in the preparation of a medicament for preventing or treating non-alcoholic fatty liver disease.

2. The use according to claim 1, wherein the Wuling formulation comprises, in parts by weight: 200 to 400 parts of Bupleuri radix, 100 to 200 parts of Ganoderma, 200 to 350 parts of Salviae miltiorrhizae radix et rhizome and 250 to 350 parts of Schisandrae fructus.

3. The use according to claim 2, wherein the Wuling formulation comprises, in parts by weight: 300 to 350 parts of Bupleuri radix, 150 to 180 parts of Ganoderma, 300 to 350 parts of Salviae miltiorrhizae radix et rhizome and 300 to 350 parts of Schisandrae fructus.

4. The use according to claim 3, wherein the Wuling formulation comprises, in parts by weight: 342 parts of Bupleuri radix, 173 parts of Ganoderma, 342 parts of Salviae miltiorrhizae radix et rhizome and 342 parts of Schisandrae fructus.

5. The use according to any one of claims 1 to 4, wherein the Wuling formulation is prepared in a dosage form selected from the group consisting of a decoction, a pill, an oral liquid, a tablet, a capsule, a granule, a medicated electuary, and a powder.

6. The use according to claim 5, wherein the Wuling formulation is selected from any one of Wuling Capsule, Wuling Pill and Wuling Powder.

7. The use according to any one of claims 1 to 6, wherein the Wuling formulation is administered to an adult in a unit dose of 3 g to 6 g, three times per day.

Example Image

Normal Control

Model Control

Atorvastatin Calcium 11.6 µg/mL

Wuling Capsule 12.5 µg/mL

Wuling Capsule 25.0 µg/mL

Wuling Capsule 50.0 µg/mL

Figure 1

Figure 2

**EP 4 751 728 A1**

## INTERNATIONAL SEARCH REPORT

| International application No. |
|---|
| **PCT/CN2024/104919** |

### A. CLASSIFICATION OF SUBJECT MATTER

A61K36/79(2006.01)i; A61K36/57(2006.01)i; A61K36/233(2006.01)i; A61P1/16(2006.01)i; A61P3/06(2006.01)i

According to International Patent Classification (IPC) or to both national classification and IPC

### B. FIELDS SEARCHED

Minimum documentation searched (classification system followed by classification symbols)

IPC,CPC:A61K,A61P

Documentation searched other than minimum documentation to the extent that such documents are included in the fields searched

Electronic data base consulted during the international search (name of data base and, where practicable, search terms used)

CJFD, CNTXT, ENTXTC, CNKI, 百度学术, BAIDU SCHOLAR, 读秀, DUXIU: 清华德人, 杜成强, 非酒精, 肝, 脂肪肝, 脂肪性肝炎, 五灵, 胶囊, 制剂, 柴胡, 丹参, 灵芝, 五味子, non alcoholic fatty liver disease, fatty liver, liver, traditional Chinese medicine, capsule, preparation, bupleuri, Salvia miltiorrhiza, Ganoderma lucidum, Schisandra chinensis

### C. DOCUMENTS CONSIDERED TO BE RELEVANT

| Category* | Citation of document, with indication, where appropriate, of the relevant passages | Relevant to claim No. |
|---|---|---|
| PX | CN 118045133 A (QINGHUA DEREN XI'AN XINGFU PHARMACEUTICAL CO., LTD.) 17 May 2024 (2024-05-17) claims 1-7 | 1-7 |
| Y | CN 112345679 A (QINGHUA DEREN XI'AN XINGFU PHARMACEUTICAL CO., LTD.) 09 February 2021 (2021-02-09) description, paragraph [0002] | 1-7 |
| Y | 唐开斌等 (TANG, Kaibin et al.). "健脾降脂方联合五灵肝复胶囊治疗脾虚浊瘀内阻型非酒精性脂肪性肝炎的临床疗效 (The Clinical Effect of Jianpi Jiangzhi Decoction Combined with Wuling Fugan Capsule in the Treatment of Spleen Beficiency Turbidity and Blood Stasis Resistance Type Nonalcoholic Fatty Hepatitis)" 现代生物医学进展 (Progress in Modern Biomedicine). Vol. 15, No. 08, 20 March 2015 (2015-03-20), pages 1495-1497 and 1588 abstract, and page 1497, left-hand column, paragraph 2 | 1-7 |
| Y | CN 101474254 A (FOURTH MILITARY MEDICAL UNIVERSITY OF CHINESE PEOPLE'S LIBERATION ARMY) 08 July 2009 (2009-07-08) description, page 2, paragraphs 1-16 | 1-7 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **05 September 2024** | **11 September 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/104919** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 110064005 A (XI'AN BOAI PHARMACEUTICAL CO., LTD.) 30 July 2019 (2019-07-30)<br>description, paragraphs [0006]-[0030] | 1-7 |
| A | CN 101837049 A (XI'AN BOAI PHARMACEUTICAL CO., LTD.) 22 September 2010 (2010-09-22)<br>description, paragraphs [0007]-[0033] | 1-7 |
| A | KR 20190059531 A (GUO XIANGWEI) 31 May 2019 (2019-05-31)<br>abstract | 1-7 |
| A | 瞿新红等 (QU, Xinhong et al.). "正交试验探讨五灵胶囊配伍对慢性肝损伤小鼠的影响 (Non-official translation: Orthogonal Test to Explore the Effect of Wuling Capsule Compatibility on Mice with Chronic Liver Injury)"<br>*中成药 (Chinese Traditional Patent Medicine),*<br>Vol. 33, No. 02, 20 February 2011 (2011-02-20), pages 232-235<br>abstract, and pages 234-235, section 3 | 1-7 |

Form PCT/ISA/210 (second sheet) (July 2022)

## INTERNATIONAL SEARCH REPORT
### Information on patent family members

International application No.

**PCT/CN2024/104919**

| Patent document cited in search report | | | Publication date (day/month/year) | Patent family member(s) | Publication date (day/month/year) |
|---|---|---|---|---|---|
| CN | 118045133 | A | 17 May 2024 | None | |
| CN | 112345679 | A | 09 February 2021 | None | |
| CN | 101474254 | A | 08 July 2009 | None | |
| CN | 110064005 | A | 30 July 2019 | None | |
| CN | 101837049 | A | 22 September 2010 | None | |
| KR | 20190059531 | A | 31 May 2019 | None | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310919939
- CN 202410263883

- FR 7909